(19) Europäisches Patentamt / European Patent Office / Office européen des brevets

(11) **EP 3 896 446 A1**

(12) **EUROPEAN PATENT APPLICATION**
published in accordance with Art. 153(4) EPC

(43) Date of publication:
**20.10.2021 Bulletin 2021/42**

(21) Application number: **19895273.1**

(22) Date of filing: **12.12.2019**

(51) Int Cl.:
*G01N 33/44* (2006.01)      *A61F 13/84* (2006.01)
*C08J 3/12* (2006.01)

(86) International application number:
**PCT/JP2019/048810**

(87) International publication number:
**WO 2020/122210 (18.06.2020 Gazette 2020/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.12.2018  JP 2018232857**
**22.03.2019  JP 2019055280**

(71) Applicant: **Sumitomo Seika Chemicals Co., Ltd.**
**Kako-gun, Hyogo 675-0145 (JP)**

(72) Inventors:
• **YABUGUCHI, Hiroki**
**Himeji-shi, Hyogo 672-8076 (JP)**
• **TSURU, Haruka**
**Himeji-shi, Hyogo 672-8076 (JP)**

(74) Representative: **Nederlandsch Octrooibureau**
**P.O. Box 29720**
**2502 LS The Hague (NL)**

(54) **METHOD FOR EVALUATING LIQUID LEAKAGE FROM WATER-ABSORBING RESIN PARTICLES**

(57)    Disclosed is a method for evaluating liquid leakage properties of water-absorbing resin particles. The method includes, in the following order: disposing a water-absorbing layer formed from the water-absorbing resin particles along an inclined plane inclined with respect to a horizontal plane; injecting a test solution which is in the form of liquid droplets and contains water into the water-absorbing layer exposed; and measuring a diffusion distance which is a distance in which the test solution injected into the water-absorbing layer diffuses along the inclined plane in a predetermined time. An angle of inclination formed by the horizontal plane and the inclined plane is 25 degrees or larger and 40 degrees or smaller.

*Fig.1*

**Description**

**Technical Field**

The present invention relates to a method for evaluating liquid leakage properties of water-absorbing resin particles.

**Background Art**

Conventionally, an absorbent containing water-absorbing resin particles has been used in an absorbent article for absorbing a liquid containing water such as urine as a main component. In general, it has been strongly desired to suppress occurrence of liquid leakage from an absorbent article during its use as much as possible (for example, Patent Literature 1, Patent Literature 2, and Patent Literature 3).

**Citation List**

**Patent Literature**

[Patent Literature 1] Japanese Unexamined Patent Publication No. 2007-69161
[Patent Literature 2] Japanese Unexamined Patent Publication No. 2006-167480
[Patent Literature 3] Japanese Unexamined Patent Publication No. 2013-63254

**Summary of Invention**

**Technical Problem**

An aspect of the present invention provides a method for evaluating, with high accuracy, a degree of occurrence of liquid leakage from an absorbent article when water-absorbing resin particles are used for the absorbent article. Another aspect of the present invention provides water-absorbing resin particles that can effectively contribute to suppressing occurrence of liquid leakage from the absorbent article.

**Solution to Problem**

An aspect of the present invention provides a method for evaluating liquid leakage properties of water-absorbing resin particles. The "liquid leakage properties of water-absorbing resin particles" referred to herein means a degree to which a liquid that contains water and is not absorbed by the water-absorbing resin particles is generated when the liquid comes into contact with an assemblage of the water-absorbing resin particles. The method according to an aspect of the present invention includes, in the following order: disposing a water-absorbing layer formed from the water-absorbing resin particles along an inclined plane inclined with respect to a horizontal plane; injecting a test solution which is in the form of liquid droplets and contains water into the water-absorbing layer; and measuring a diffusion distance which is a distance in which the test solution injected into the water-absorbing layer diffuses along the inclined plane in a predetermined time. An angle of inclination formed by the horizontal plane and the inclined plane is 40 degrees or smaller. The angle of inclination may be 25 degrees or larger and 40 degrees or smaller.
Another aspect of the present invention relates to water-absorbing resin particles in which a diffusion distance measured by the above method is shorter than 14 cm. The diffusion distance referred to herein is measured under the following conditions: the angle of inclination formed by the horizontal plane and the inclined plane is 30 degrees; the test solution is an aqueous solution containing sodium chloride at a concentration of 0.780% by mass, calcium chloride at a concentration of 0.022% by mass, magnesium sulfate at a concentration of 0.038% by mass, and ion exchange water, where the concentrations are concentrations based on a mass of the aqueous solution; the test solution at 25°C which is in the form of liquid droplets is injected into the water-absorbing layer in an amount of 0.25 mL; and the predetermined time is 30 seconds from a timing when the test solution is injected into the water-absorbing layer.

**Advantageous Effects of Invention**

According to an aspect of the present invention, it is possible to provide a method for evaluating, with high accuracy, a degree of occurrence of liquid leakage from an absorbent article when water-absorbing resin particles are used for the absorbent article. According to another aspect of the present invention, it is possible to provide water-absorbing resin particles that can effectively contribute to suppressing occurrence of liquid leakage from the absorbent article.

**Brief Description of Drawings**

Fig. 1 is a schematic view showing an embodiment of a method for evaluating liquid leakage properties of water-absorbing resin particles.
Fig. 2 is a plan view showing an embodiment of a support plate and a water-absorbing layer.
Fig. 3 is a cross-sectional view showing an embodiment of an absorbent article.
Fig. 4 is a schematic view showing a method for evaluating liquid leakage properties of the absorbent article.

**Description of Embodiments**

Hereinafter, some embodiments of the present invention will be described in detail. However, the present invention is not limited to the following embodiments.

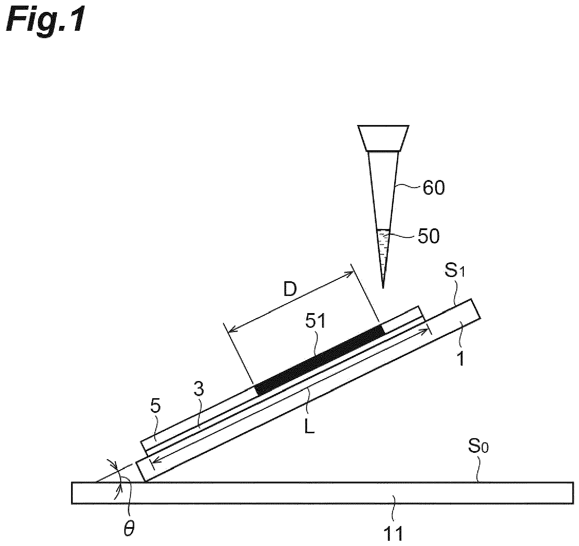

Fig. 1 is a schematic view showing an embodiment of a method for evaluating liquid leakage properties of water-absorbing resin particles. The method shown in Fig. 1 includes, in the following order: disposing a water-absorbing layer 5 formed from water-absorbing resin particles along an inclined plane $S_1$ inclined with respect to a horizontal plane $S_0$; injecting a test solution 50 in the form of liquid droplets into the water-absorbing layer 5; and measuring a diffusion distance D which is a distance in which the test solution 51 injected into the water-absorbing layer 5 diffuses along the inclined plane $S_1$ in a predetermined time. An angle of inclination $\theta$ formed by the horizontal plane $S_0$ and the inclined plane $S_1$ is 40 degrees or smaller. According to the findings of the inventors of the present invention, the diffusion distance D measured by this method shows a favorable correlation with a degree of occurrence of liquid leakage from an absorbent article manufactured by using the water-absorbing resin particles.

The water-absorbing layer 5 is formed by fixing the water-absorbing resin particles onto an adhesive surface of an adhesive tape 3. The water-absorbing layer 5 can be formed, for example, by a method of dispersing the water-absorbing resin particles on the adhesive surface of the adhesive tape 3, and pressing the dispersed water-absorbing resin particles against the adhesive surface with a roller. A more specific example is as follows: the water-absorbing resin particles are dispersed on an adhesive surface having a length L of 15 cm and a width of 5 cm, a stainless steel roller (with a diameter of 10.5 cm and a width of 6.0 cm), which has a mass of 4.0 kg and is placed on the dispersed water-absorbing resin particles, is moved, and thereby the water-absorbing resin particles are pressed against the adhesive surface. A thickness of the water-absorbing layer 5 may be $1.0 \pm 0.2$ mm.

A support plate 1 having a flat main surface is prepared. The support plate 1 is fixed on a board 11 having a horizontal plane $S_0$ in a direction such that the main surface of the support plate 1 becomes an inclined plane $S_1$ inclined with respect to the horizontal plane $S_0$. The support plate 1 can be fixed by using a general instrument such as a clamp. The support plate 1 is not particularly limited as long as it is smooth, but it may be, for example, an acrylic resin plate. The adhesive tape 3 onto which the water-absorbing layer 5 is fixed is bonded to the inclined plane $S_1$ of the fixed support plate 1.

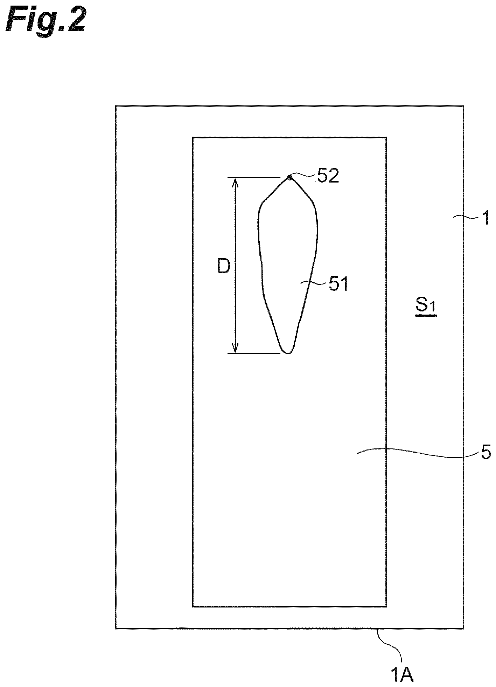

Fig. 2 is a plan view of the support plate 1 and the water-absorbing layer 5 of Fig. 1 which are seen from a direction perpendicular to the inclined plane $S_1$. The test solution 51 injected into the water-absorbing layer 5 diffuses along the inclined plane $S_1$ in a manner of mainly flowing down the inclined plane $S_1$. The diffusion distance D is a distance in which the injected test solution 51 diffuses in a predetermined time from an injection point 52 at which the test solution 50 in the form of liquid droplets has been injected. The diffusion distance D is defined as a maximum moving distance of the test solution 51 from the injection point 52, in a direction perpendicular to a line of intersection between the inclined plane $S_1$ and the horizontal plane $S_0$. In the case of Fig. 2, a short side 1A of the main surface (inclined plane $S_1$) of the support plate 1 generally corresponds to the line of intersection between the inclined plane $S_1$ and the horizontal plane $S_0$.

The diffusion distance D is measured when a predetermined time has elapsed from a timing when the test solution 50 has been injected into the water-absorbing layer 5. The predetermined time referred to herein can be appropriately adjusted within a range in which the diffusion distance D can be appropriately measured. Specifically, the predetermined time for measuring the diffusion distance D may be, for example, 10 seconds or longer, or 20 seconds or longer, and it may be, for example, 120 seconds or shorter, or 60 seconds or shorter. The predetermined time for measuring the diffusion distance D may be 10 seconds or longer and 120 seconds or shorter or 60 seconds or shorter, or may be 20 seconds or longer and 120 seconds or shorter or 60 seconds or shorter.

The test solution 50 contains water. Components and a concentration of the test solution 50 can be selected in consideration of usage applications and the like of an absorbent article for which the water-absorbing resin particles are used. The test solution 50 may be an aqueous solution containing metal ions such as sodium ions, or may be artificial urine obtained by quasi-reproduction of urine. An example of the test solution 50 is an aqueous solution (artificial urine) sodium chloride at a concentration of 0.780% by mass, calcium chloride at a concentration of 0.022% by mass, magnesium sulfate at a concentration of 0.038% by mass, and water. The concentration of each of the component referred to herein is a concentration based on a mass of the aqueous solution (artificial urine). The test solution 50 may be colored by adding an appropriate colorant or the like so that the diffusion distance D can be easily distinguished.

A temperature of the test solution 50 injected into the water-absorbing layer 5 may be 20°C to 40°C, or 25°C. In general, the water-absorbing layer 5 is also disposed in the environment of the same temperature.

A predetermined amount of the test solution 50 added dropwise from a pipette 60 disposed above the water-absorbing layer 5 is injected into the water-absorbing layer 5 at one time. As the pipette 60, it is possible to use means, such as a micropipette, capable of quantitatively determining a dropping amount. An amount of the test solution 50 injected is adjusted within a range in which the diffusion distance D can be appropriately measured. For example, an amount of the test solution 50 injected at one time may be 0.1 to 1.0 mL, 0.2 to 0.8 mL, 0.2 to 0.5 mL, or 0.25 mL.

Then angle of inclination $\theta$ formed by the horizontal plane $S_0$ and the inclined plane $S_1$ is larger than 0 degrees and 40 degrees or smaller. When the angle of inclination $\theta$ is 40 degrees or smaller, the diffusion distance D easily reflects characteristics of the water-absorbing resin particles, and thereby it is possible to accurately and easily evaluate liquid leakage properties of the water-absorbing resin particles. From the same viewpoint, the angle of inclination $\theta$ may be 10 degrees or larger, 20 degrees or larger, or 25 degrees or larger.

The water-absorbing resin particles according to an embodiment show a diffusion distance D of shorter than 14 cm when evaluated by the method described above. The diffusion distance D referred to herein is measured under the following conditions: an angle of inclination $\theta$ is 30 degrees; the test solution 50 is an aqueous solution containing sodium chloride at a concentration of 0.780% by mass, calcium chloride at a concentration of 0.022% by mass, magnesium sulfate at a concentration of 0.038% by mass, and water; the test solution 50 at 25°C which is in the form of liquid droplets is injected into the water-absorbing layer 5 in an amount of 0.25 mL; and a predetermined time at which the diffusion distance D is measured is 30 seconds from a timing when the test solution 50 is injected into the water-absorbing layer 5.

By using the water-absorbing resin particles showing a diffusion distance D of shorter than 14 cm measured by the method under the above conditions, it is possible to easily obtain an absorbent article in which occurrence of leakage of urine or the like is sufficiently suppressed. From the same viewpoint, a diffusion distance D of the water-absorbing resin particles may be 11 cm or shorter, 10 cm or shorter, or 8 cm or shorter. A diffusion distance D thereof may be 0 cm or longer, or 1 cm or longer.

Examples of shapes of the water-absorbing resin particles according to the present embodiment include a spherical shape, a crushed shape, and a granular shape. A median particle size of the water-absorbing resin particles according to the present embodiment may be 250 to 850 $\mu$m, 300 to 700 $\mu$m, or 300 to 600 $\mu$m. The water-absorbing resin particles according to the present embodiment may have a desired particle size distribution at a timing obtained in a production method to be described later, but their particle size distribution may be adjusted by performing operations such as adjustment of a particle size through classification with a sieve. The median particle size can be measured by a method disclosed in WO2018/181548.

The water-absorbing resin particles according to the present embodiment can contain, for example, a crosslinked polymer formed by polymerization of monomers including ethylenically unsaturated monomers. The crosslinked polymer has one or two or more monomer units derived from an ethylenically unsaturated monomer. As the ethylenically unsaturated monomer, a water-soluble ethylenically unsaturated monomer can be used. In the present specification, the term "water-soluble" means that a solubility of 5% by mass or more is exhibited in water at 25°C. The phrase "5% by mass or more" referred to herein is a value of concentration based on a mass of water.

The water-absorbing resin particles can be produced by a method including a step of polymerizing monomers including ethylenically unsaturated monomers. Examples of methods of polymerization include a reverse-phase suspension polymerization method, an aqueous solution polymerization method, a bulk polymerization method, and a precipitation polymerization method. The reverse-phase suspension polymerization method or the aqueous solution polymerization method may be adopted from the viewpoints of facilitating securement of favorable water absorption characteristics of the obtained water-absorbing resin particles and control of a polymerization reaction. Hereinbelow, a method for polymerizing ethylenically unsaturated monomers will be described with the reverse-phase suspension polymerization method as an example.

An ethylenically unsaturated monomer may be water-soluble. Examples of water-soluble ethylenically unsaturated monomers include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where an ethylenically unsaturated monomer has an amino group, the amino group may be quatemarized. The ethylenically unsaturated monomer may be used alone or in a combination of two or more kinds thereof. A functional group, such as a carboxyl group and an amino group, of the monomer, may function as a crosslinkable functional group in a surface crosslinking process to be described later.

Among them, from the viewpoint of high industrial availability, the ethylenically unsaturated monomer may include at least one compound selected from the group consisting of (meth)acrylic acid and a salt thereof, acrylamide, methacrylamide, and N,N-dimethyl acrylamide. The ethylenically unsaturated monomer may include at least one compound selected from the group consisting of (meth)acrylic acid and a salt thereof, and acrylamide. The ethylenically unsaturated monomer may include at least one compound selected from the group consisting of (meth)acrylic acid and a salt thereof from the viewpoint of further enhancing water absorption characteristics.

For the monomer for obtaining the water-absorbing resin particles, a monomer other than the above-described ethylenically unsaturated monomers may be used. Such a monomer can be used by, for example, being mixed with an aqueous solution containing the above-described ethylenically unsaturated monomers. A proportion of the ethylenically unsaturated monomers may be 70 to 100 mol% with respect to a total amount of monomers. A proportion of (meth)acrylic acid and a salt thereof may be 70 to 100 mol% with respect to a total amount of monomers.

Usually, the ethylenically unsaturated monomers are suitably used in a form of an aqueous solution. A concentration of the ethylenically unsaturated monomers in an aqueous solution containing the ethylenically unsaturated monomers (hereinafter, simply referred to as an "aqueous solution of monomers") may be 20% by mass or more and a saturated concentration or less, 25% to 70% by mass, or 30% to 55% by mass. Examples of water to be used an aqueous solution include tap water, distilled water, and ion exchange water.

In the aqueous solution of monomers, in a case where ethylenically unsaturated monomers have an acidic group, the ethylenically unsaturated monomers may be polymerized after neutralizing this acidic group with an alkaline neutralizing agent. From the viewpoint of increasing an osmotic pressure of the obtained water-absorbing resin particles and thereby further enhancing water absorption characteristics (such as a water retention capacity), a degree of neutralization in the ethylenically unsaturated monomers by the alkaline neutralizing agent may be 10 to 100 mol%, 50 to 90 mol%, or 60 to 80 mol% of the acidic group in the ethylenically unsaturated monomers. Examples of alkaline neutralizing agents include alkali metal salts such as sodium hydroxide, sodium carbonate, sodium hydrogen carbonate, potassium hydroxide, and potassium carbonate; and ammonia. The alkaline neutralizing agent may be used alone or in combination of two or more kinds thereof. The alkaline neutralizing agent may be used in a form of an aqueous solution to simplify a neutralizing operation. Neutralization of the acidic groups in the ethylenically unsaturated monomers can be performed by, for example, adding an aqueous solution of sodium hydroxide, potassium hydroxide, or the like dropwise to the above-described aqueous solution of monomers and mixing them.

In the reverse-phase suspension polymerization method, an aqueous solution of monomers can be dispersed in a hydrocarbon dispersion medium in the presence of a surfactant, and polymerization of ethylenically unsaturated monomers can be performed using a radical polymerization initiator or the like. The radical polymerization initiator may be a water-soluble radical polymerization initiator.

Examples of surfactants include nonionic surfactants and anionic surfactants. Examples of nonionic surfactants include sorbitan fatty acid esters, (poly)glycerin fatty acid esters (where "(poly)" means both of a case with the prefix "poly" and a case without the prefix "poly," and the same applies hereinbelow), sucrose fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene hydrogenated castor oil, alkylallyl formaldehyde condensed polyoxyethylene ethers, polyoxyethylene polyoxypropylene block copolymers, polyoxyethylene polyoxypropyl alkyl ethers, and polyethylene glycol fatty acid esters. Examples of anionic surfactants include fatty acid salts, alkylbenzene sulfonate, alkylmethyl taurate, polyoxyethylene alkylphenyl ether sulfuric acid ester salts, polyoxyethylene alkyl ether sulfonic acid salts, phosphoric acid esters of polyoxyethylene alkyl ethers, and phosphoric acid esters of polyoxyethylene alkyl allyl ethers. The surfactant may be used alone or in combination of two or more kinds thereof.

The surfactant may include at least one compound selected from the group consisting of sorbitan fatty acid esters, polyglycerin fatty acid esters, and sucrose fatty acid esters, from the viewpoints that then, a state of a W/O type reverse-phase suspension becomes favorable, water-absorbing resin particles having a suitable particle size are easily obtained, and industrial availability becomes high. The surfactant may include sucrose fatty acid esters or may include sucrose stearic acid esters from the viewpoint that water absorption characteristics of the obtained water-absorbing resin particles are then easily improved.

A usage amount of the surfactant may be 0.05 to 10 parts by mass, 0.08 to 5 parts by mass, or 0.1 to 3 parts by mass, with respect to 100 parts by mass of the aqueous solution of monomers, from the viewpoint that a sufficient effect is obtained within these usage amounts, and these amounts are economic.

In the reverse-phase suspension polymerization, a polymeric dispersant may be used in combination with the above-mentioned surfactant. Examples of polymeric dispersants include maleic anhydride-modified polyethylene, maleic anhydride-modified polypropylene, a maleic anhydride-modified ethylene-propylene copolymer, a maleic anhydride-modified EPDM (ethylene propylene diene terpolymer), maleic anhydride-modified polybutadiene, a maleic anhydride-ethylene copolymer, a maleic anhydride-propylene copolymer, a maleic anhydride-ethylene-propylene copolymer, a maleic anhydride-butadiene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, ethyl hydroxyethyl cellulose, and the like. The polymeric dispersant may be used alone or in combination of two or more kinds thereof. From the viewpoint of better dispersion stability of monomers, the polymeric dispersant may include at least one selected from the group consisting of maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene, and an oxidized ethylene-propylene copolymer.

A usage amount of the polymeric dispersant may be 0.05 to 10 parts by mass, 0.08 to 5 parts by mass, or 0.1 to 3 parts by mass, with respect to 100 parts by mass of the aqueous solution of monomers, from the viewpoint that a sufficient effect is obtained within these usage amounts, and these amounts are economic.

The hydrocarbon dispersion medium may include at least one compound selected from the group consisting of a chained aliphatic hydrocarbon having 6 to 8 carbon atoms and an alicyclic hydrocarbon having 6 to 8 carbon atoms. Examples of hydrocarbon dispersion media include chained aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. The hydrocarbon dispersion medium may be used alone or in combination of two or more kinds thereof.

For the hydrocarbon dispersion medium, at least one selected from the group consisting of n-heptane and cyclohexane may be contained from the viewpoints of high industrial availability and stable qualities. Furthermore, from the same viewpoints, as a mixture of the hydrocarbon dispersion media, for example, a commercially available Exxsol Heptane (manufactured by ExxonMobil Chemical: containing n-heptane and 75% to 85% of hydrocarbons of isomers thereof) may be used.

A usage amount of the hydrocarbon dispersion medium may be 30 to 1,000 parts by mass, 40 to 500 parts by mass, or 50 to 300 parts by mass, with respect to 100 parts by mass of the aqueous solution of monomers, from the viewpoint that polymerization heat is then appropriately removed, and thereby a polymerization temperature is easily controlled. In a case where a usage amount of the hydrocarbon dispersion medium is 30 parts by mass or more, there is a tendency that it becomes easy to control a polymerization temperature. In a case where a usage amount of the hydrocarbon dispersion medium is 1,000 parts by mass or less, there is a tendency that productivity of polymerization is improved, which is economic.

A radical polymerization initiator may be water-soluble. Examples of water-soluble radical polymerization initiators include persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis[2-(2-imidazolin-2-yl)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). The radical polymerization initiator may be used alone or in combination of two or more kinds thereof. The radical polymerization initiator may include at least one selected from the group consisting of potassium persulfate, ammonium persulfate, sodium persulfate, 2,2'-azobis(2-amidinopropane) dihydrochloride, 2,2'-azobis[2-(2-imidazolin-2-yl)propane] dihydrochloride, and 2,2'-azobis {2-[1-(2-hydroxyethyl)-2-imidazolin-2-yl]propane} dihydrochloride.

A usage amount of the radical polymerization initiator may be 0.00005 to 0.01 moles with respect to 1 mole of the ethylenically unsaturated monomers. A case in which a usage amount of the radical polymerization initiator is 0.00005 moles or more is efficient, because then a polymerization reaction is not required to be performed for a long period of time. In a case where a usage amount of the radical polymerization initiator is 0.01 moles or less, it is easy to suppress occurrence of a rapid polymerization reaction.

The radical polymerization initiator can also be used as a redox polymerization initiator when it is used in combination with a reducing agent such as sodium sulfite, sodium hydrogen sulfite, ferrous sulfate, and L-ascorbic acid.

In a polymerization reaction, an aqueous solution of monomers used for the polymerization may contain a chain transfer agent. Examples of chain transfer agents include hypophosphites, thiols, thiolic acids, secondary alcohols, and amines.

The aqueous solution of monomers used for the polymerization may contain a thickener to control a particle size of the water-absorbing resin particles. Examples of thickeners include hydroxyethyl cellulose, hydroxypropyl cellulose, methyl cellulose, carboxymethyl cellulose, polyethylene glycol, polyacrylamide, polyethyleneimine, dextrin, sodium alginate, polyvinyl alcohol, polyvinylpyrrolidone, polyethylene oxide, and the like. In a case where stirring speeds in the polymerization are the same, a median particle size of particles to be obtained is likely to become large as a viscosity of the aqueous solution of monomers becomes high.

Crosslinking may occur by self-crosslinking upon the polymerization, but crosslinking may be carried out by further using an internal crosslinking agent. By using the internal crosslinking agent, it is easy to control water absorption characteristics of the water-absorbing resin particles. The internal crosslinking agent is generally added to a reaction solution in the polymerization reaction. Examples of internal crosslinking agents include di- or tri(meth)acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the polyols with unsaturated acids (such as maleic acid and fumaric acid); bis(meth)acrylamides such as N,N'-methylenebis(meth)acrylamide; di- or tri(meth)acrylic acid esters obtained by reacting a polyepoxide with (meth)acrylic acid; carbamyl di(meth)acrylate esters obtained by reacting a polyisocyanate (such as tolylene diisocyanate and hexamethylene diisocyanate) with hydroxyethyl (meth)acrylate; compounds having two or more polymerizable unsaturated groups, such as allylated starch, allylated cellulose, diallyl phthalate, N, N', N"-triallyl isocyanurate, and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromohydrin, and $\alpha$-methyl epichlorohydrin; compounds having two or more reactive functional groups, such as isocyanate compounds (such as 2,4-tolylene diisocyanate and hexamethylene diisocyanate); and the like. The internal crosslinking agent may be used alone or in combination of two or more kinds thereof. The internal crosslinking agent may include a polyglycidyl compound, may include a diglycidyl ether compound, or may include at least one selected from the group consisting of (poly)ethylene glycol diglycidyl ether, (poly)propylene glycol diglycidyl ether, and (poly)glycerin diglycidyl ether.

A usage amount of the internal crosslinking agent may be 0 mmol or more, 0.02 mmol or more, 0.03 mmol or more, 0.04 mmol or more, or 0.05 mmol or more, and may be 0.1 moles or less, per 1 mole of the ethylenically unsaturated monomer, from the viewpoints of suppressing water-soluble properties by appropriately crosslinking the obtained polymer, and easily obtaining a sufficient water absorption capacity. In particular, in first-stage polymerization in multi-stage reverse-phase suspension polymerization, when an amount of the internal crosslinking agent is 0.03 mmol or more per 1 mole of the ethylenically unsaturated monomer, it is easy to obtain water-absorbing resin particles showing a short diffusion distance D.

An aqueous phase containing an ethylenically unsaturated monomer, a radical polymerization initiator, and if necessary, an internal crosslinking agent, and the like; and an oil phase containing a hydrocarbon dispersant, and if necessary, a surfactant and a polymeric dispersant, and the like can be heated under stirring in a state where they are mixed to carry out reverse-phase suspension polymerization in a water-in-oil system.

When performing the reverse-phase suspension polymerization, an aqueous solution of monomers which contains ethylenically unsaturated monomers is dispersed in a hydrocarbon dispersion medium in the presence of a surfactant (and if necessary, a polymeric dispersant). In this case, a timing of adding the surfactant, the polymeric dispersant, or the like before the start of the polymerization reaction may be either before or after the addition of the aqueous solution of monomers.

The polymerization may be carried out after dispersing the aqueous solution of monomers in the hydrocarbon dispersion medium in which the polymeric dispersant has been dispersed, and then further dispersing the surfactant in the hydrocarbon dispersion medium, from the viewpoint that an amount of the hydrocarbon dispersion medium remaining in the obtained water-absorbing resin can then be easily reduced.

The reverse-phase suspension polymerization can be carried out in one stage or in multiple stages of two or more stages. The reverse-phase suspension polymerization may be carried out in two or three stages from the viewpoint of increasing productivity.

In a case where reverse-phase suspension polymerization is carried out in multiple stages of two or more stages, it is sufficient for stages after a second stage of reverse-phase suspension polymerization to be carried out in the same manner as in a first stage of reverse-phase suspension polymerization by adding ethylenically unsaturated monomers to a reaction mixture obtained in the first stage of polymerization reaction and mixing them, after performing the first stage of reverse-phase suspension polymerization. In reverse-phase suspension polymerization in each stage after the second stage, reverse-phase suspension polymerization may be carried out by adding, in addition to ethylenically unsaturated monomers, the above-mentioned radical polymerization initiator and/or internal crosslinking agent within a range of molar ratios of the respective components to the ethylenically unsaturated monomers, based on an amount of ethylenically unsaturated monomers added during reverse-phase suspension polymerization in each stage after the second stage. If necessary, the internal crosslinking agent may be used in reverse-phase suspension polymerization in each stage after the second stage. In a case where the internal crosslinking agent is used, reverse-phase suspension polymerization may be carried out by adding the internal crosslinking agent within a range of molar ratios of the respective components to the ethylenically unsaturated monomers based on an amount of ethylenically unsaturated monomers provided in each stage.

A temperature for the polymerization reaction varies depending on radical polymerization initiators used, and it may be 20°C to 150°C, or 40°C to 120°C, from the viewpoint that the polymerization is then promptly performed, which shortens a polymerization time, and thereby economic efficiency increases, and that polymerization heat is then easily removed, and thereby the reaction is smoothly performed. A reaction time is generally 0.5 to 4 hours. Completion of the polymerization reaction can be confirmed from, for example, stop of temperature rising in the reaction system. Accordingly, a polymer of ethylenically unsaturated monomers is generally obtained in a state of a hydrous gel polymer.

After the polymerization, post-polymerization crosslinking may be carried out by adding a crosslinking agent to the obtained hydrous gel polymer and heating them. By performing the post-polymerization crosslinking, a degree of crosslinking of the hydrous gel polymer can be increased, and thereby water absorption characteristics of the water-absorbing resin particles can be further improved.

Examples of crosslinking agents for performing the post-polymerization crosslinking include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups, such as (poly)ethylene glycol diglycidyl ether, (poly)propylene glycol diglycidyl ether, and (poly)glycerin diglycidyl ether; haloepoxy compounds such as epichlorohydrin, epibromohydrin, and α-methyl epichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis[N,N-di(β-hydroxyethyl)]adipamide. The crosslinking agent for the post-polymerization crosslinking may be polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether. These crosslinking agents may be used alone or in combination of two or more kinds thereof.

An amount of the crosslinking agent used in the post-polymerization crosslinking may be 0 to 0.03 moles, 0 to 0.01 moles, or 0.00001 to 0.005 moles in a molar ratio, per 1 mole of the ethylenically unsaturated monomer, from the viewpoint of exhibiting suitable water absorption characteristics by appropriately crosslinking the obtained hydrous gel polymer. When an amount of the crosslinking agent added is within the above range, it is easy to obtain water-absorbing resin particles showing a short diffusion distance D.

It is sufficient for a timing for adding the post-polymerization crosslinking to be after polymerization of ethylenically unsaturated monomers used for the polymerization. In a case of multi-stage polymerization, the crosslinking agent for the post-polymerization crosslinking may be added after the multi-stage polymerization. From the viewpoint of a water content (to be described later), it is preferable to add the crosslinking agent for the post-polymerization crosslinking within a region of [water content immediately after polymerization ± 3% by mass], in consideration of heat generation during and after polymerization, retention due to process delay, system opening when a crosslinking agent is added, and fluctuation in moisture content due to addition of water associated with addition of a crosslinking agent.

Subsequently, drying is performed to remove moisture from the obtained hydrous gel polymer. By drying, polymer particles containing the polymer of ethylenically unsaturated monomers are obtained. Examples of drying methods include a method (a) in which a hydrous gel polymer in a state of being dispersed in a hydrocarbon dispersion medium is subjected to azeotropic distillation by heating from the outside, and the hydrocarbon dispersion medium is refluxed to remove moisture; a method (b) in which a hydrous gel polymer is taken out by decantation and dried under reduced pressure; and a method (c) in which a hydrous gel polymer is separated by filtration with a filter and dried under reduced pressure. Among them, the method (a) may be generally used for its simplicity in a production process.

It is possible to adjust a particle size of the water-absorbing resin particles by adjusting a rotational speed of a stirrer during the polymerization reaction or by adding a flocculating agent to the system after the polymerization reaction or at an initial time of drying. A particle size of the obtained water-absorbing resin particle can be increased by adding the flocculating agent. As the flocculating agent, an inorganic flocculating agent can be used. Examples of inorganic flocculating agents (for example, powdery inorganic flocculating agents) include silica, zeolite, bentonite, aluminum oxide, talc, titanium dioxide, kaolin, clay, and hydrotalcite. The flocculating agent may include at least one selected from the group consisting of silica, aluminum oxide, talc, and kaolin from the viewpoint of a flocculation effect.

In the reverse-phase suspension polymerization, a method of adding the flocculating agent may be a method in which a flocculating agent is dispersed in a hydrocarbon dispersion medium of the same kind as that used in the polymerization, or water in advance, and then the mixture is mixed into a hydrocarbon dispersion medium containing a hydrous gel polymer under stirring.

An amount of the flocculating agent added may be 0.001 to 1 part by mass, 0.005 to 0.5 parts by mass, or 0.01 to 0.2 parts by mass, with respect to 100 parts by mass of ethylenically unsaturated monomers used in the polymerization. By setting an amount of the flocculating agent added to be within the above range, it is easy to obtain water-absorbing resin particles having a desired particle size distribution.

In the production of the water-absorbing resin particles, a surface portion of the hydrous gel polymer is preferably crosslinked (surface-crosslinked) using a crosslinking agent in the drying process or any of subsequent processes. By performing surface crosslinking, it is easy to control water absorption characteristics of the water-absorbing resin particles.

The surface crosslinking may be performed at a timing when the hydrous gel polymer has a specific water content. A timing of the surface crosslinking may be a time point at which a water content of the hydrous gel polymer is 5% to 50% by mass, a time point at which a water content thereof is 10% to 40% by mass, or a time point at which a water content thereof is 15% to 35% by mass. A water content (% by mass) of the hydrous gel polymer is calculated by the following formula.

$$\text{Water content} = [Ww/(Ww + Ws)] \times 100$$

Ww: An amount of water of a hydrous gel polymer which is a value obtained by adding an amount of water used, as desired, upon mixing a flocculating agent, a surface crosslinking agent, and the like to an amount obtained by subtracting an amount of water extracted to the outside of the system by the drying process from an amount of water contained in an aqueous solution of monomers before polymerization in the all polymerization processes.
Ws: A solid fraction calculated from an amount of materials introduced, such as ethylenically unsaturated monomers, a crosslinking agent, and an initiator, each of which constitutes the hydrous gel polymer.

Examples of crosslinking agents (surface crosslinking agents) for performing surface crosslinking include compounds having two or more reactive functional groups. Examples of crosslinking agents include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and $\alpha$-methyl epichlorohydrin; isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; hydroxyalkylamide compounds such as bis[N,N-di($\beta$-hydroxyethyl)]adipamide; and the like. The crosslinking agent may be used alone or in combination of two or more kinds thereof. The crosslinking agent may include a polyglycidyl compound. The crosslinking agent may include at least one selected from the group consisting of (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether.
In general, a usage amount of the surface crosslinking agent may be 0.00001 to 0.02 moles, 0.00005 to 0.01 moles, or 0.0001 to 0.005 moles, with respect to 1 mole of the ethylenically unsaturated monomer used in the polymerization, from the viewpoint of exhibiting suitable water absorption characteristics by appropriately crosslinking the obtained hydrous gel polymer. When an amount of the surface crosslinking agent added is within the above range, it is easy to obtain water-absorbing resin particles showing a short diffusion distance D.
It is possible to obtain polymer particles, which are a surface-crosslinked dried product, by distilling off water and the hydrocarbon dispersion medium by a known method after the surface crosslinking.
The water-absorbing resin particles according to the present embodiment may be composed of only the polymer particles, but they can further contain, for example, various additional components selected from gel stabilizers, metal chelating agents (ethylenediaminetetraacetic acid and its salts, diethylenetriaminepentaacetic acid and its salts, for example, diethylenetriaminepentaacetic acid pentasodium, and the like), and flowability improvers (lubricants). The additional components may be disposed inside the polymer particles, on a surface of the polymer particles, or both of the inside and on the surface thereof. The additional component may be flowability improvers (lubricants). A flowability improver may be inorganic particles. Examples of inorganic particles include silica particles such as amorphous silica.
The water-absorbing resin particles may contain a plurality of inorganic particles disposed on the surface of the polymer particles. The inorganic particles can be disposed on the surface of the polymer particles by, for example, mixing the polymer particles and the inorganic particles. These inorganic particles may be silica particles such as amorphous silica. In a case where the water-absorbing resin particles contain inorganic particles disposed on the surface of the polymer particles, a ratio of the inorganic particles to a mass of the polymer particles may be 0.2% by mass or more, 0.5% by mass or more, 1.0% by mass or more, or 1.5% by mass or more, and it may be 5.0% by mass or less or 3.5% by mass or less. A ratio of the inorganic particles to a mass of the polymer particles may be 0.2% by mass or more and 5.0% by mass or less or 3.5% by mass or less, may be 0.5% by mass or more and 5.0% by mass or less or 3.5% by mass or less, may be 1.0% by mass or more and 5.0% by mass or less or 3.5% by mass or less, or may be 1.5% by mass or more and 5.0% by mass or less or 3.5% by mass or less. The inorganic particles referred to herein generally have a minute size as compared with a size of the polymer particles. For example, an average particle size of the inorganic particles may be 0.1 to 50 $\mu$m, 0.5 to 30 $\mu$m, or 1 to 20 $\mu$m. The average particle size referred to herein can be a value measured by a dynamic light scattering method or a laser diffraction/scattering method. By setting an amount of the inorganic particles added to be within the above range, it is easy to obtain water-absorbing resin particles showing a short diffusion distance D among water absorption characteristics of the water-absorbing resin particles.

An embodiment of the method for producing water-absorbing resin particles may further include a step of evaluating liquid leakage properties of the obtained water-absorbing resin particles by the method according to the above-described embodiment. For example, water-absorbing resin particles, in which a measurement value of a diffusion distance D is smaller than a reference value (for example, 14 cm), may be sorted out. Accordingly, it is possible to more stably produce the water-absorbing resin particles capable of suppressing liquid leakage from an absorbent article.

An absorbent according to an embodiment contains the water-absorbing resin particles according to the present embodiment. The absorbent according to the present embodiment can contain a fibrous material, and it is, for example, a mixture containing the water-absorbing resin particles and the fibrous material. The configuration of the absorbent may be, for example, a configuration in which water-absorbing resin particles and the fibrous materials are uniformly mixed, a configuration in which water-absorbing resin particles are held between fibrous materials formed in a sheet shape or a layer shape, or another configuration.

Examples of fibrous materials include finely pulverized wood pulp; cotton; cotton linter; rayon; cellulose-based fibers such as cellulose acetate; synthetic fibers such as polyamides, polyesters, and polyolefins; a mixture of these fibers; and the like. One kind of the fibrous materials may be used alone, or two or more kinds thereof may be used in combination. The fibrous material may be hydrophilic fibers.

A mass proportion of the water-absorbing resin particles in the absorbent with respect to a total amount of the water-absorbing resin particles and the fibrous material may be 2% to 100% by mass, 10% to 80% by mass, or 20% to 60% by mass.

Fibers may be adhered to each other by adding an adhesive binder to the fibrous material in order to enhance shape retention properties before or during use of the absorbent. Examples of adhesive binders include thermal bonding synthetic fibers, hot-melt adhesives, and adhesive emulsions. The adhesive binder may be used alone or in combination of two or more kinds thereof.

Examples of thermal bonding synthetic fibers include full-melt binders such as polyethylene, polypropylene, and an ethylene-propylene copolymer; partial-melt binders formed of polypropylene and polyethylene in a side-by-side or core-and-sheath configuration; and the like. In the above-mentioned partial-melt binders, only a polyethylene portion can be thermal-bonded.

A hot-melt adhesive may be a mixture of a base polymer with a viscosity imparting agent, a plasticizer, an antioxidant, or the like. The base polymer may include at least one polymer selected from the group consisting of ethylene-vinyl acetate copolymer, a styrene-isoprene-styrene block copolymer, a styrene-butadiene-styrene block copolymer, a styrene-ethylene-butylene-styrene block copolymer, a styrene-ethylene-propylene-styrene block copolymer, and an amorphous polypropylene.

Examples of adhesive emulsions include polymers of at least one monomer selected from the group consisting of methyl methacrylate, styrene, acrylonitrile, 2-ethylhexyl acrylate, butyl acrylate, butadiene, ethylene, and vinyl acetate.

The absorbent according to the present embodiment may contain an inorganic powder (for example, amorphous silica), a deodorant, an antibacterial agent, a fragrance, and the like. In a case where the water-absorbing resin particles contain inorganic particles, the absorbent may contain an inorganic powder in addition to the inorganic particles in the water-absorbing resin particles.

A shape of the absorbent according to the present embodiment is not particularly limited, but it may be, for example, a sheet shape. A thickness of the absorbent (for example, a thickness of a sheet-shaped absorbent) may be, for example, 0.1 to 20 mm or 0.3 to 15 mm.

An absorbent article according to the present embodiment includes the absorbent according to the present embodiment. Examples of members constituting the absorbent article according to the present embodiment include a core wrap that retains the shape of the absorbent; a liquid-permeable sheet disposed on the outermost part on a side from which an absorption target liquid is infiltrated; a liquid-impermeable sheet disposed on the outermost part on a side opposite to the side from which the absorption target liquid is infiltrated; and the like. Examples of the absorbent article include diapers (for example, paper diapers), toilet training pants, incontinence pads, hygiene products (sanitary napkins, tampons, and the like), sweat pads, pet sheets, portable toilet members, animal excrement treatment materials, and the like.

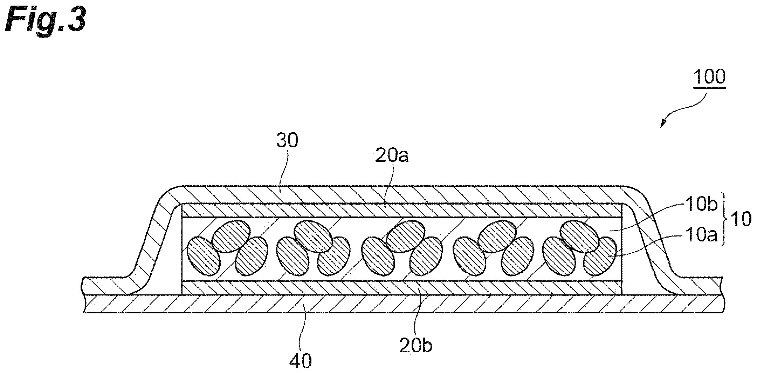

Fig. 3 is a cross-sectional view showing an example of an absorbent article. An absorbent article 100 shown in Fig. 3 includes an absorbent 10, core wraps 20a and 20b, a liquid-permeable sheet 30, and a liquid-impermeable sheet 40. In the absorbent article 100, the liquid-impermeable sheet 40, the core wrap 20b, the absorbent 10, the core wrap 20a, and the liquid-permeable sheet 30 are laminated in this order. In Fig. 3, there is a portion shown to be a gap between the members, but the members may be in close contact with each other without the gap.

The absorbent 10 has water-absorbing resin particles 10a according to the present embodiment and a fiber layer 10b containing a fibrous material. A plurality of the water-absorbing resin particles 10a is dispersed in the fiber layer 10b.

A content of the water-absorbing resin particles 10a may be 100 to 1,000 g per square meter of the absorbent 10 (that is, 100 to 1,000 g/m$^2$), 150 to 800 g/m$^2$, or 200 to 700 g/m$^2$, from the viewpoint that sufficient liquid absorption performances can then be more easily obtained when the absorbent 10 is used for the absorbent article 100. A content of the water-absorbing resin particles 10a may be 100 g/m$^2$ or more from the viewpoint of exhibiting sufficient liquid absorption performances as the absorbent article 100, and thereby particularly suppressing liquid leakage. A content of the water-absorbing resin particles 10a may be 1,000 g/m$^2$ or less from the viewpoint of suppressing occurrence of a gel blocking phenomenon, and thereby exhibiting a diffusion performance of a liquid as the absorbent article 100 and further improving a permeation speed of the liquid.

A content of the fiber layer 10b (fibrous material) may be 50 to 800 g per square meter of the absorbent 10 (that is, 50 to 800 g/m$^2$), 100 to 600 g/m$^2$, or 150 to 500 g/m$^2$, from the viewpoint that sufficient liquid absorption performances are then obtained when the absorbent 10 is used for the absorbent article 100. A content of the fiber layer 10b (fibrous material) may be 50 g or more per square meter of the absorbent 10 (that is, 50 g/m$^2$ or more) from the viewpoint of exhibiting sufficient liquid absorption performances as the absorbent article 100, and thereby particularly suppressing occurrence of a gel blocking phenomenon to improve a diffusion performance of a liquid, and increasing strength of the absorbent 10 after it absorbs a liquid. A content of the fiber layer 10b (fibrous material) may be 800 g or less per square meter of the absorbent 10 (that is, 800 g/m$^2$ or less) from the viewpoint of particularly suppressing reversion after liquid absorption.

The core wrap 20a is disposed on one surface side of the absorbent 10 (an upper side of the absorbent 10 in Fig. 3) in a state of being in contact with the absorbent 10. The core wrap 20b is disposed on the other surface side of the absorbent 10 (a lower side of the absorbent 10 in Fig. 3) in a state of being in contact with the absorbent 10. The absorbent 10 is disposed between the core wrap 20a and the core wrap 20b. Examples of the core wraps 20a and 20b include tissues, non-woven fabrics, and the like. The core wrap 20a and the core wrap 20b each have, for example, a main surface having the same size as that of the absorbent 10.

The liquid-permeable sheet 30 is disposed on the outermost part on a side from which an absorption target liquid is infiltrated. The liquid-permeable sheet 30 is disposed on the core wrap 20a in a state of being in contact with the core wrap 20a. Examples of the liquid-permeable sheet 30 include non-woven fabrics made of synthetic resin such as polyethylene, polypropylene, polyester, and polyamide, and porous sheets. The liquid-impermeable sheet 40 is disposed on the outermost part on a side opposite to the liquid-permeable sheet 30, in the absorbent article 100. The liquid-impermeable sheet 40 is disposed below the core wrap 20b in a state of being in contact with the core wrap 20b. Examples of the liquid-impermeable sheet 40 include sheets made of synthetic resins such as polyethylene, polypropylene, and polyvinyl chloride, and sheets made of a composite material of these synthetic resins and a non-woven fabric. The liquid-permeable sheet 30 and the liquid-impermeable sheet 40 each have, for example, a main surface wider than the main surface of the absorbent 10, and outer edges of the liquid-permeable sheet 30 and the liquid-impermeable sheet 40 respectively extend around the absorbent 10 and the core wraps 20a and 20b.

A magnitude relationship between the absorbent 10, the core wraps 20a and 20b, the liquid-permeable sheet 30, and the liquid-impermeable sheet 40 is not particularly limited, and it is appropriately adjusted according to usage applications and the like of the absorbent article. Furthermore, a method of retaining the shape of the absorbent 10 using the core wraps 20a and 20b is not particularly limited. The absorbent may be wrapped with a plurality of the core wraps as shown in Fig. 3, or the absorbent may be wrapped with one core wrap.

According to the present embodiment, it is possible to provide a method of absorbing a liquid using the water-absorbing resin particles, absorbent, or absorbent article according to the present embodiment. The method of absorbing a liquid according to the present embodiment includes a step of bringing an absorption target liquid into contact with the water-absorbing resin particles, absorbent, or absorbent article according to the present embodiment.

Examples

Hereinafter, the present invention will be more specifically described with reference to examples. However, the present invention is not limited to these examples.

Examination 1

1-1. Production of water-absorbing resin particles

Example 1

First-stage polymerization reaction

A cylindrical round-bottomed separable flask was prepared, which had an inner diameter of 11 cm and an internal capacity of 2 L, and was equipped with a reflux condenser, a dropping funnel, a nitrogen gas introduction tube, and as a stirrer, a stirring blade having four inclined paddle blades, each having a blade diameter of 5 cm, in a two-tier manner. 293 g of n-heptane, and 0.736 g of a maleic anhydride-modified ethylene-propylene copolymer (HI-WAX 1105A, manufactured by Mitsui Chemicals, Inc.) as polymeric dispersant were put into this flask. The reaction solution in the flask was heated to 80°C while being stirred to dissolve the polymeric dispersant in the n-heptane. Thereafter, the reaction solution was cooled to 50°C.

92.0 g (1.03 moles) of aqueous solution of acrylic acid at a concentration of 80.5% by mass was put into a beaker with an internal capacity of 300 mL. 147.7 g of aqueous solution of sodium hydroxide at a concentration of 20.9% by mass was added dropwise into the aqueous solution of acrylic acid while cooling the beaker from the outside, and thereby 75 mol% of acrylic acid was neutralized. Then, 0.092 g of hydroxylethyl cellulose (HEC AW-15F, manufactured by Sumitomo Seika Chemicals Co., Ltd.) as a thickener, 0.092 g (0.339 mmol) of 2,2'-azobis(2-amidinopropane) dihydrochloride and 0.018 g (0.068 mmol) of potassium persulfate as a radical polymerization initiator, and 0.010 g (0.057 mmol) of ethylene glycol diglycidyl ether as an internal crosslinking agent were dissolved in the aqueous solution of acrylic acid. Thereby, a first-stage aqueous monomer solution was prepared.

The first-stage aqueous monomer solution was added into the above-mentioned reaction solution in the separable flask, and the reaction solution was stirred for 10 minutes. Then, a surfactant solution containing 6.62 g of n-heptane and 0.736 g of sucrose stearic acid ester (HLB: 3, manufactured by Mitsubishi-Chemical Foods Corporation, RYOTO Sugar Ester S-370) was added into the reaction solution. While stirring the reaction solution at 550 rpm as a rotational speed of the stirring blade, the inside of the system was sufficiently replaced with nitrogen. Thereafter, a polymerization reaction was caused to proceed for 60 minutes while heating the separable flask in a water bath at 70°C. By this polymerization reaction, a first-stage polymerization slurry liquid containing a hydrous gel polymer was obtained.

Second-stage polymerization reaction

128.8 g (1.43 moles) of aqueous solution of acrylic acid at a concentration of 80.5% by mass was put into a beaker with an internal capacity of 500 mL. 159.0 g of aqueous solution of sodium hydroxide at a concentration of 27% by mass was added dropwise into the aqueous solution of acrylic acid while cooling the beaker from the outside, and thereby 75 mol% of acrylic acid was neutralized. Then, 0.129 g (0.475 mmol) of 2,2'-azobis(2-amidinopropane) dihydrochloride and 0.026 g (0.095 mmol) of potassium persulfate were dissolved in the aqueous solution of acrylic acid. Thereby, a second-stage aqueous solution of monomers was prepared.

While stirring the first-stage polymerization slurry liquid in the separable flask at 1,000 rpm as a rotational speed of the stirring blade, the separable flask was cooled to 25°C. Then, a total amount of the second-stage aqueous solution of monomers was added thereinto, and then the inside of the system was replaced with nitrogen over 30 minutes. Thereafter, a polymerization reaction was caused to proceed for 60 minutes while heating the separable flask in a water bath at 70°C. Thereafter, 0.580 g (0.067 mmol) of an aqueous solution of ethylene glycol diglycidyl ether at a concentration of 2% by mass was added as a crosslinking agent for post-polymerization crosslinking.

0.265 g of aqueous solution of diethylenetriaminepentaacetic acid pentasodium at a concentration of 45% by mass was added into the reaction solution containing the hydrous gel polymer under stirring. Thereafter, the flask was immersed in an oil bath set to 125°C, and 238.5 g of water was extracted out of the system while n-heptane was refluxed by azeotropic distillation with n-heptane and water. Thereafter, 4.42 g (0.507 mmol) of aqueous solution of 2% by mass ethylene glycol diglycidyl ether as a surface crosslinking agent was added into the flask, and maintained at 83°C for 2 hours.

From the reaction solution obtained after the surface crosslinking reaction, n-heptane was distilled off by heating at 125°C to obtain polymer particles (dried product). The obtained polymer particles were passed through a sieve having an aperture of 850 $\mu$m. Thereafter, 2.0% by mass of amorphous silica (Oriental Silicas Corporation, Tokusil NP-S) with respect to a mass of the polymer particles was mixed with the polymer particles, and thereby 232.2 g of water-absorbing resin particles containing the amorphous silica was obtained. A median particle size of the water-absorbing resin particles was 393 $\mu$m.

Example 2

Polymer particles (dried product) were obtained in the same procedure as in Example 1 except that, in the first-stage polymerization reaction, a radical polymerization initiator was changed to 0.0736 g (0.272 mmol) of potassium persulfate, but 2,2'-azobis(2-amidinopropane) dihydrochloride was not used; and in the second-stage polymerization reaction, a radical polymerization initiator was changed to 0.090 g (0.334 mmol) of potassium persulfate, but 2,2'-azobis(2-amidinopropane) dihydrochloride was not used. An amount of water extracted from a reaction solution containing a hydrous gel polymer was 247.9 g.

The obtained polymer particles were passed through a sieve having an aperture of 850 $\mu$m. Thereafter, 0.5% by mass of amorphous silica (Oriental Silicas Corporation, Tokusil NP-S) with respect to a mass of the polymer particles was mixed with the polymer particles, and thereby 231.0 g of water-absorbing resin particles was obtained. A median particle size of the water-absorbing resin particles was 355 μm.

Comparative Example 1

A polyacrylic acid 250,000 (average molecular weight: about 250,000) manufactured by Wako Pure Chemical Industries, Ltd. was prepared as water-absorbing resin particles of Comparative Example 1. A median particle size of the water-absorbing resin particles was 343 μm.

1-2. Test for evaluating leakage properties

(1) Preparation of artificial urine

Sodium chloride, calcium chloride, and magnesium sulfate, each at the following concentration, were dissolved in ion exchange water. A small amount of Blue No. 1 was added to the obtained solution to obtain artificial urine colored in blue. The obtained artificial urine was used as a test solution for evaluating leakage properties. The following concentrations are concentrations based on a total mass of the artificial urine.
Composition of artificial urine

NaCl: 0.780% by mass
$CaCl_2$: 0.022% by mass
$MgSO_4$: 0.038% by mass
Blue No. 1: 0.002% by mass

(2) Test Method-1 (water-absorbing resin particles)

Liquid leakage properties of the water-absorbing resin particles were evaluated by the following procedures i), ii), iii), iv) and v).

i) A strip-shaped adhesive tape (manufactured by DIATEX Co., Ltd., PYOLAN tape) having a length of 15 cm and a width of 5 cm was put on a laboratory table such that an adhesive surface faced up, and 3.0 g of the water-absorbing resin particles was evenly dispersed onto this adhesive surface. A stainless steel roller (mass 4.0 kg, diameter 10.5 cm, width 6.0 cm) was placed on the upper part of the dispersed water-absorbing resin particles, and this roller was caused to reciprocate three times between both ends of the adhesive tape in a longitudinal direction. Accordingly, a water-absorbing layer formed from the water-absorbing resin particles was formed on the adhesive surface of the adhesive tape.
ii) The adhesive tape was stood vertically to remove excess water-absorbing resin particles from the water-absorbing layer. The roller was placed on the water-absorbing layer again and caused to reciprocate three times between both ends of the adhesive tape in the longitudinal direction.
iii) In a room at a temperature of 25 ± 2°C, an acrylic resin plate having a rectangular flat main surface with a length of 30 cm and a width of 55 cm was fixed such that its width direction was parallel to the horizontal plane, and an angle between its main surface and the horizontal plane was 10 degrees, 20 degrees, 30 degrees, or 40 degrees (hereinafter also referred to as an inclined plane). The adhesive tape on which the water-absorbing layer was formed was bonded onto the inclined plane of the fixed acrylic plate such that the water-absorbing layer was exposed and in a direction in which the longitudinal direction of the adhesive tape was perpendicular to the width direction of the acrylic resin plate.
iv) Using a micropipette (PIPETMAN Neo P1000N manufactured by M&S Instruments Inc.), 0.25 mL of a test solution at a liquid temperature of 25°C was entirely injected within 1 second into a position about 1 cm from the upper end of the water-absorbing layer, from a height of about 1 cm from the surface.
v) 30 seconds after the start of the injection of the test solution, a maximum value of a moving distance of the test solution injected into the water-absorbing layer was read and recorded as a diffusion distance D. The diffusion distance D is a distance on the inclined plane connecting a dropping point (injection point) and the longest reaching point of the test solution with a straight line in a direction perpendicular to the short side of the acrylic resin plate.

(3) Test Method-2 (absorbent article)

10 g of the water-absorbing resin particles and 9.5 g of pulverized pulp were uniformly mixed by air papermaking, and thereby a sheet-shaped absorbent having a size of 12 cm × 32 cm was produced. The absorbent was placed on a core wrap (tissue paper), and a core wrap (tissue paper) and a liquid-permeable sheet were placed on the absorbent. A load of 588 kPa was applied for 30 seconds to the absorbent sandwiched between the core wrap and the liquid-permeable sheet. Furthermore, a polyethylene liquid-impermeable sheet having a size of 12 cm $\times$ 32 cm was bonded to a surface on the side opposite to the liquid-permeable sheet, and thereby an absorbent article for testing was obtained.

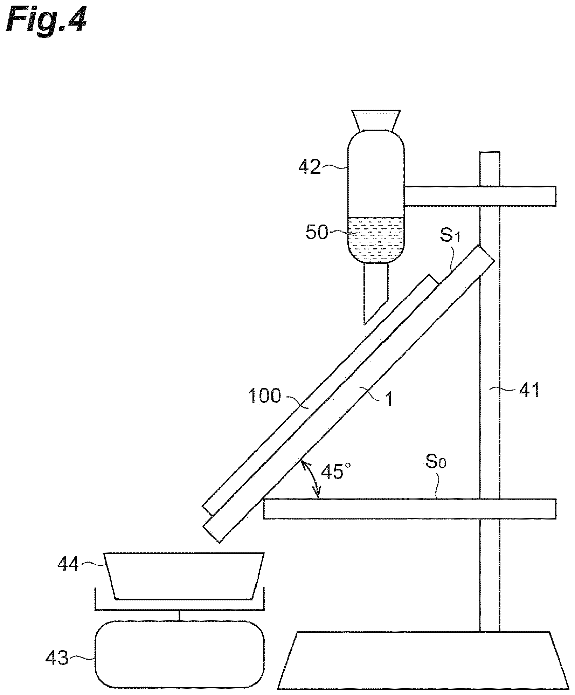

Fig. 4 is a schematic view showing a method for evaluating leakage properties of an absorbent article. A support plate 1 (herein referred to as an acrylic resin plate) with a length of 45 cm and having a flat inclined plane $S_1$ was fixed by a stand 41 in a state of being inclined at $45 \pm 2$ degrees with respect to a horizontal plane $S_0$. An absorbent article 100 for testing was bonded onto the inclined plane $S_1$ of the fixed support plate 1 in a room at a temperature of $25 \pm 2°C$ such that a longitudinal direction of the absorbent article 100 was along a longitudinal direction of the support plate 1. Next, a test solution 50 (artificial urine) adjusted to $25 \pm 1°C$ was added dropwise from a dropping funnel 42 vertically disposed above the absorbent article toward a position 8 cm, in an upper direction, from the center of an absorbent in the absorbent article 100. 80 mL of the test solution was added dropwise at a time at a speed of 8 mL/sec. A distance between a tip end of the dropping funnel 42 and the absorbent article was $10 \pm 1$ mm. The test solution was repeatedly added under the same conditions at intervals of 10 minutes from the start of the first addition of the test solution. The test solution was added 5 times in total.

In a case where the test solution that was not absorbed by the absorbent article 100 leaked out from a lower part of the support plate 1, the leaked test solution was recovered in a metal tray 44 disposed below the support plate 1. A weight (g) of the recovered test solution was measured by a balance 43, and this value was recorded as an amount of leakage. In the case of Comparative Example 1, the test was completed at a timing of the second addition in which a large amount of leakage occurred. The amount of leakage was subtracted from a total amount of the test solution added to calculate an amount of absorption until the leakage occurred. It is judged that as this numerical value becomes large, liquid leakage is unlikely to occur when the absorbent article is worn.

(4) Results

The evaluation results are shown in Table 1. A diffusion distance D of "14\*" in Test Method-1 indicates that the test solution leaked from the lower part of the water-absorbing layer. Based on the results of Test Method-2, it can be said that the water-absorbing resin particles of Examples 1 and 2 can provide an absorbent article in which occurrence of leakage is suppressed, as compared with the water-absorbing resin particles of Comparative Example 1. In a case where an angle of inclination θ was 45 degrees in Test Method-1, a diffusion distance D longer than 14 cm was shown, and the test solution leaked from the lower part of the water-absorbing layer in both Example 2 and Comparative Example 1, and therefore it is impossible to predict a difference in leakage properties of the absorbent articles based on these results. On the other hand, it can be seen that when an angle of inclination θ was 40 degrees or smaller, a diffusion distance D shows a favorable correlation with leakage properties of the absorbent articles.

Table 1

| | Test Method-1 (water-absorbing resin particles) | | | | | Test Method-2 (absorbent article) | | | | | | Amount of absorption until leakage occurs [g] |
|---|---|---|---|---|---|---|---|---|---|---|---|---|
| | Diffusion distance D [cm] | | | | | Amount of leakage [g] | | | | | | |
| | 10 deg. | 20 deg. | 30 deg. | 40 deg. | 50 deg. | 1st | 2nd | 3rd | 4th | 5th | Total | |
| Ex. 1 | 3.0 | 4.0 | 7.5 | 9.0 | 10.5 | 0 | 0 | 0 | 0 | 0 | 0 | 400 |
| Ex. 2 | 2.0 | 4.0 | 9.0 | 13.0 | 14* | 0 | 0 | 0 | 0 | 7 | 7 | 393 |
| Comp. Ex. 1 | 1.0 | 9.0 | 14* | 14* | 14* | 0 | 62 | - | - | - | 62 | 98 |

Examination 2

2-1. Production of water-absorbing resin particles

Example 3

229.2 g of water-absorbing resin particles containing 0.5% by mass of amorphous silica with respect to a mass of polymer particles (dried product) was obtained in the same procedure as in Example 2. However, an amount of water extracted out of the system from a reaction solution containing a hydrous gel polymer by azeotropic distillation was 239.7 g. A median particle size of the water-absorbing resin particles was 377 $\mu$m.

Example 4

232.1 g of water-absorbing resin particles was obtained in the same procedure as in Example 1 except that an amount of amorphous silica was changed to 0.2% by mass with respect to a mass of polymer particles (dried product). A median particle size of the water-absorbing resin particles was 396 $\mu$m.

Comparative Example 2

Polymer particles (dried product) were obtained in the same procedure as in Example 1 except that, in the first-stage polymerization reaction, a radical polymerization initiator was changed to 0.0736 g (0.272 mmol) of potassium persulfate, but 2,2'-azobis(2-amidinopropane) dihydrochloride was not used; in the second-stage polymerization reaction, a radical polymerization initiator was changed to 0.090 g (0.334 mmol) of potassium persulfate, but 2,2'-azobis(2-amidinopropane) dihydrochloride was not used; in the second-stage polymerization reaction, 0.0116 g (0.067 mmol) of ethylene glycol diglycidyl ether was used as an internal crosslinking agent; and a crosslinking agent for post-polymerization crosslinking was not added. An amount of water extracted from a reaction solution containing a hydrous gel polymer by azeotropic distillation was 256.1 g.

The obtained polymer particles were passed through a sieve having an aperture of 850 $\mu$m. Thereafter, 0.1% by mass of amorphous silica (Oriental Silicas Corporation, Tokusil NP-S) with respect to a mass of the polymer particles of the water-absorbing resin particles was mixed with the polymer particles, and thereby 230.8 g of water-absorbing resin particles was obtained. A median particle size of the water-absorbing resin particles was 349 $\mu$m.

Comparative Example 3

Polymer particles (dried product) were obtained in the same procedure as in Example 1 except that, in the first-stage polymerization reaction, an amount of ethylene glycol diglycidyl ether as an internal crosslinking agent was changed to 0.0046 g (0.026 mmol); in the second-stage polymerization reaction, 0.0116 g (0.067 mmol) of ethylene glycol diglycidyl ether was used as an internal crosslinking agent; a crosslinking agent for post-polymerization crosslinking was not added; and an amount of an aqueous solution of ethylene glycol diglycidyl ether at a concentration of 2% by mass as a surface crosslinking agent was changed to 6.62 g (0.761 mmol). An amount of water extracted from a reaction solution containing a hydrous gel polymer by azeotropic distillation was 219.2 g.

The obtained polymer particles were passed through a sieve having an aperture of 850 $\mu$m. Thereafter, 0.20% by mass of amorphous silica (Oriental Silicas Corporation, Tokusil NP-S) with respect to a mass of the polymer particles was mixed with the polymer particles, and thereby 229.6 g of water-absorbing resin particles was obtained. A median particle size of the water-absorbing resin particles was 356 $\mu$m.

Comparative Example 4

Polymer particles (dried product) were obtained in the same procedure as in Example 1 except that, in the first-stage polymerization reaction, an amount of ethylene glycol diglycidyl ether as an internal crosslinking agent was changed to 0.0046 g (0.026 mmol); in the second-stage polymerization reaction, 0.0116 g (0.067 mmol) of ethylene glycol diglycidyl ether was used as an internal crosslinking agent; and a crosslinking agent for post-polymerization crosslinking was not added. An amount of water extracted from a reaction solution containing a hydrous gel polymer by azeotropic distillation was 234.2 g.

The obtained polymer particles were passed through a sieve having an aperture of 850 $\mu$m. Thereafter, 0.20% by mass of amorphous silica (Oriental Silicas Corporation, Tokusil NP-S) with respect to a mass of the polymer particles was mixed with the polymer particles, and thereby 229.6 g of water-absorbing resin particles was obtained. A median particle size of the water-absorbing resin particles was 355 $\mu$m.

2-2. Test for evaluating liquid leakage properties

Liquid leakage properties of the water-absorbing resin particles and the absorbent article was evaluated by Test Method-1 and Test Method-2. An angle of inclination $\theta$ in Test Method-1 was fixed at 30 degrees. Table 2 collectively shows the evaluation results of Examples 1 to 4 and Comparative Examples 2 to 4. A diffusion distance D of "14*" in Test Method-1 indicates that the test solution leaked from the lower part of the water-absorbing layer. A diffusion distance obtained by Test Method-1 showed a favorable correlation with leakage properties of the absorbent article. In addition, it was also confirmed that the water-absorbing resin particles of Examples 1 to 4 in which a diffusion distance was shorter than 14 cm could provide the absorbent articles in which occurrence of leakage was sufficiently suppressed.

Table 2

| | Test Method-1 (water - absorbing resin particles) | Test Method-2 (absorbent article) | | | | | | Amount of absorption until leakage occurs [g] |
|---|---|---|---|---|---|---|---|---|
| | Diffusion distance D [cm] | Amount of leakage [g] | | | | | | |
| | 30 degrees | 1st | 2nd | 3rd | 4th | 5th | Total | |
| Ex. 1 | 7.5 | 0 | 0 | 0 | 0 | 0 | 0 | 400 |
| Ex. 2 | 9.0 | 0 | 0 | 0 | 0 | 7 | 7 | 393 |
| Ex. 3 | 10.0 | 0 | 0 | 0 | 0 | 3 | 3 | 397 |
| Ex. 4 | 12.0 | 0 | 0 | 0 | 0 | 26 | 26 | 374 |
| Comp. Ex. 2 | 14* | 0 | 0 | 0 | 6 | 43 | 49 | 314 |
| Comp. Ex. 3 | 14* | 0 | 0 | 0 | 21 | 12 | 33 | 299 |
| Comp. Ex. 4 | 14* | 0 | 0 | 0 | 8 | 35 | 43 | 312 |

**Reference Signs List**

1: support plate, 3: adhesive tape, 5: water-absorbing layer, 10: absorbent, 10a: water-absorbing resin particle, 10b: fiber layer, 20a, 20b: core wrap, 30: liquid-permeable sheet, 40: liquid-impermeable sheet, 50: test solution, 51: test solution injected into water-absorbing layer, 60: pipette, 100: absorbent article, $S_0$: horizontal plane, $S_1$: inclined plane, 0: angle of inclination

**Claims**

1.  A method for evaluating liquid leakage properties of water-absorbing resin particles, the method comprising, in the following order:

    disposing a water-absorbing layer formed from the water-absorbing resin particles along an inclined plane inclined with respect to a horizontal plane;
    injecting a test solution which is in the form of liquid droplets and contains water into the water-absorbing layer exposed; and
    measuring a diffusion distance which is a distance in which the test solution injected into the water-absorbing layer diffuses along the inclined plane in a predetermined time,
    wherein an angle of inclination formed by the horizontal plane and the inclined plane is 25 degrees or larger and 40 degrees or smaller, and
    the water-absorbing layer fixed on an adhesive tape is formed by dispersing 3.0 g of the water-absorbing resin particles on a rectangular adhesive surface, which has a length of 15 cm and a width of 5 cm, of the adhesive tape, and moving a stainless steel roller with a diameter of 10.5 cm and a width of 6.0 cm, which has a mass of 4.0 kg and is placed on the dispersed water-absorbing resin particles, to press the water-absorbing resin particles against the adhesive surface; and the water-absorbing layer is disposed along the inclined plane by bonding the adhesive tape on which the water-absorbing layer is fixed to the inclined plane.

2.  A method for producing water-absorbing resin particles, the method comprising: evaluating liquid leakage properties of the water-absorbing resin particles by the method according to claim 1.

*Fig.1*

**Fig.2**

**Fig.3**

# Fig.4

<table>
<tr><td colspan="2">**INTERNATIONAL SEARCH REPORT**</td><td colspan="2">International application No.<br>PCT/JP2019/048810</td></tr>
</table>

**A. CLASSIFICATION OF SUBJECT MATTER**
G01N 33/44(2006.01)i; A61F 13/84(2006.01)i; C08J 3/12(2006.01)i
FI: G01N33/44; C08J3/12 Z CEY; A61F13/84 100

According to International Patent Classification (IPC) or to both national classification and IPC

**B. FIELDS SEARCHED**

Minimum documentation searched (classification system followed by classification symbols)
G01N33/44; A61F13/15-13/84; C08J3/12; C08F20/04-20/06; C08F120/04-120/06;
C08F220/04-220/06

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2017/170501 A1 (NIPPON SHOKUBAI CO., LTD.) 05.10.2017 (2017-10-05) paragraphs [0251]-[0266], fig. 6 | 1, 2 |
| A | JP 2008-178667 A (KAO CORP.) 07.08.2008 (2008-08-07) paragraph [0117] | 1, 2 |
| A | JP 2003-088552 A (SUMITOMO SEIKA CHEMICALS CO., LTD.) 25.03.2003 (2003-03-25) paragraphs [0080]-[0084], [0089]-[0091] | 1, 2 |

☐ Further documents are listed in the continuation of Box C. ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 19 February 2020 (19.02.2020) | 03 March 2020 (03.03.2020) |

| Name and mailing address of the ISA/<br>Japan Patent Office<br>3-4-3, Kasumigaseki, Chiyoda-ku,<br>Tokyo 100-8915, Japan | Authorized officer |
|---|---|
| | Telephone No. |

Form PCT/ISA/210 (second sheet) (January 2015)

International application No.

PCT/JP2019/048810

| Patent Documents referred in the Report | Publication Date | Patent Family | Publication Date |
|---|---|---|---|
| WO 2017/170501 A1 | 05 Oct. 2017 | US 2019/0125921 A1 paragraphs [0402]-[0418], fig. 6<br>EP 3437729 A1<br>KR 10-2018-0127437 A<br>CN 109070052 A | |
| JP 2008-178667 A | 07 Aug. 2008 | (Family: none) | |
| JP 2003-088552 A | 25 Mar. 2003 | (Family: none) | |

Form PCT/ISA/210 (patent family annex) (January 2015)

**EP 3 896 446 A1**

**Patent documents cited in the description**

- JP 2007069161 A
- JP 2006167480 A
- JP 2013063254 A
- WO 2018181548 A